# FASCICULE DE BREVET EUROPEEN

(11) **EP 1 301 148 B1**
(45) Date de publication et mention de la délivrance du brevet: **21.07.2004**
(21) Numéro de dépôt: 01936557.6
(22) Date de dépôt: 17.05.2001
(51) Int. Cl.: A61F 2/42, A61B 17/15, A61B 17/02, A61B 17/28

(54) **PROTHESE D'ARTICULATION**
GELENKPROTHESE
ARTICULATION PROSTHESIS

(30) Priorité: 22.05.2000 FR 0006492
(43) Date de publication de la demande: 16.04.2003
(73) Titulaire: Transystème S.A., 30000 Nimes (FR)
(72) Inventeur: ASENCIO, Joseph-Guy, F-30000 Nîmes (FR)
(74) Mandataire: Ravina, Bernard
(86) Numéro de dépôt international: PCT/FR2001/001509
(87) Numéro de publication internationale: WO 2001/089427

(56) Documents cités:
- EP-A- 0 241 846
- EP-A- 0 800 803
- WO-A-95/09588
- DE-A- 2 830 354
- FR-A- 2 220 235
- FR-A- 2 663 838
- FR-A- 2 676 917
- FR-A- 2 715 557
- FR-A- 2 730 157
- FR-A- 2 759 900
- US-A- 5 019 081
- US-A- 5 458 648
- US-A- 5 766 259

## Description

L'invention est du domaine des prothèses médicales, et plus particulièrement des prothèses d'articulation entre deux os, prothèse de la cheville interposée entre le tibia et l'astragale notamment. L'invention a aussi pour objet des instruments pour l'implantation d'une telle prothèse.

On connaît des prothèses de la cheville comprenant un élément tibial et un élément astragalien, entre lesquels est interposé un élément intermédiaire, mobile par rapport à l'élément astragalien et immobilisé par rapport à l'élément tibial. L'élément tibial est constitué d'un organe allongé épaulé. La partie allongée, dite quille, est de conformation globalement conique pour pénétrer axialement à l'intérieur d'un logement du tibia opéré par le praticien, en vue du centrage de l'élément tibial. L'épaulement est constitué par un plateau sur lequel repose le tibia, le plateau reposant lui-même sur l'élément intermédiaire. On notera qu'une fenêtre frontale est pratiquée dans le tibia pour faciliter la mise en place de l'élément tibial. L'élément astragalien est constitué d'un corps de section longitudinale globalement conformée en segment circulaire. La face inférieure du corps est pourvue d'un organe en saillie, tel qu'une quille ou des rails, d'emboîtement à l'intérieur d'un logement de l'astrale opéré par le praticien, en vue du centrage de l'élément astragalien. La face supérieure du corps est convexe pour l'appui de la face inférieure conformée en correspondance de l'élément intermédiaire. L'interposition de l'élément intermédiaire entre les éléments tibial et astragalien préalablement positionnés sur l'os y relatif, provoque leur mise en compression en appui axial contre l'os correspondant et de ce fait le maintien de l'assemblage des différents éléments de la prothèse les uns par rapport aux autres. Une telle prothèse de la cheville est notamment décrite par le document FR2220235 (ANVAR).

Un problème à résoudre réside dans la mise en place et le positionnement par le praticien des différents éléments sur les os. Plus particulièrement, une première difficulté réside dans le centrage des éléments à l'intérieur de l'os y relatif et les uns par rapport aux autres. Il en découle que les logements pratiqués extemporanément doivent rigoureusement correspondre aux organes d'emboîtement respectifs des éléments tibial et astragalien, faute de quoi il peut en résulter un mauvais fonctionnement de la prothèse.

Un autre problème à résoudre réside dans le fait qu'à l'usage les éléments de la prothèse peuvent s'avérer être inadaptés, et qu'une nouvelle intervention soit nécessaire pour un remplacement des éléments initialement mis en place. Enfin, en raison de la conformation même de l'astragale, l'organe de centrage de l'élément astragalien ne peut être étendu vers l'intérieur de l'os à l'instar de celui de l'élément tibial. Il en découle une fragilité de la tenue générale de la prothèse.

On relèvera à titre indicatif qu'il est aussi connu dans le domaine des prothèses; celles plus particulièrement destinées aux articulations de la main, notamment entre des phalanges voisines. L'anatomie des phalanges étant effilée, il a été proposé de désaxer et d'épointer la quille que porte un élément correspondant de la prothèse par rapport à l'axe général d'extension de cette dernière, pour favoriser l'insertion de cette quille à l'intérieur d'un métacarpe par un agencement de la prothèse en relation avec l'anatomie de la main. On pourra par exemple se reporter au document WO9509588 (ORTHOMET) qui décrit une telle prothèse.

Le but de la présente invention est de proposer une prothèse d'articulation entre deux os, prothèse de la cheville notamment, permettant de faciliter les opérations de préparation des os et de mise en place des éléments de la prothèse qui leur sont affectés. Un autre but de l'invention est de proposer des instruments pour la préparation de l'astragale en vue de l'implantation d'une telle prothèse. Un but visé aussi par l'invention est de faciliter l'interchangeabilité entre les différents éléments composant la prothèse. Enfin, un dernier but de l'invention est d'améliorer la compétitivité des prothèses du genre.

La prothèse d'articulation entre deux os de la présente invention, prothèse de la cheville notamment, est du genre de prothèse comprenant deux éléments d'emboîtement prévus pour être introduits à l'intérieur d'un logement d'un os respectif, pratiqué extemporanément par le praticien, et un élément intermédiaire interposé entre les éléments d'emboîtement pour leur articulation l'un par rapport à l'autre et leur mise en compression contre l'os correspondant. Les éléments d'emboîtement comportent chacun un organe distal de positionnement à l'intérieur de l'os y relatif et un organe proximal d'appui contre une face opposée respective de l'élément intermédiaire. L'organe distal de positionnement, dit quille, de l'un des éléments d'emboîtement, dit tibial dans le cas d'exemple où l'os correspondant est le tibia, est un organe de centrage d'une conformation allongée globalement conique se réduisant vers son extrémité distale, prévu pour pénétrer axialement à l'intérieur de l'os correspondant, tibia notamment. L'organe proximal d'appui de l'élément tibial contre la face correspondante de l'organe intermédiaire est quant à lui conformé en plateau, relié à l'extrémité proximale de la quille avec une orientation générale sensiblement perpendiculaire à l'axe d'extension de cette dernière.

Selon un premier aspect de l'invention, celle-ci a consisté à pourvoir la quille de l'élément tibial de moyens, dits de prise, pour sa prise et son extraction par le praticien hors du logement qui la reçoit. Ces moyens comprennent les caractéristiques suivantes, prises seules ou en combinaison :
1) La quille comporte une zone médiane d'inflexion vers son extrémité distale pour former une surface, dite de prise, inclinée par rapport à la surface extérieure d'extension générale de la quille depuis son extrémité proximale. Ces dispositions sont telles que le praticien peut prendre fermement appui contre la surface de prise au moyen d'un outil, et exercer une traction sur la quille vers son extrémité proximale en vue de son extraction hors du logement qui la reçoit.
2) La surface de prise comporte au moins une conformation globalement plane, formée par au moins une facette ménagée sur la surface extérieure de la quille. Plus particulièrement, cette facette au moins est ménagée sur une partie de la surface extérieure de la quille, dite frontale, prévue pour être disposée en vis à vis sur la fenêtre tibiale opérée par le praticien.
3) La face de liaison de la quille avec le plateau et l'axe général d'extension de la quille forment ensemble un angle inférieur à 90°, compris préférentiellement entre 80° et 88°. En d'autres termes, la quille comporte une inclinaison générale par rapport au plateau, de manière à accroître l'inclinaison de la surface de prise par rapport à l'orientation générale de ce dernier. On relèvera par ailleurs que l'inclinaison de la quille facilite son basculement à travers la fenêtre tibiale lors de la mise en place ou de l'extraction de l'élément tibial.
4) La quille comporte une conformation tronc pyramidale, de section globalement triangulaire, trapézoïdale ou analogue, à arrêtes arrondies. Ces dispositions sont telles que la quille est immobilisée en rotation grâce à sa section non circulaire, le centrage de l'élément tibial s'effectuant sans risque de flottement grâce à son positionnement au moyen de surfaces latérales globalement planes de la quille.

Selon une forme avantageuse de réalisation, les moyens de liaison entre la quille et le plateau sont des moyens de liaison par emboîtement, constitués par un organe mâle et un organe femelle coopérant, respectivement ménagés sur l'un quelconque du plateau et de la quille. Ces dispositions sont telles que le praticien peut, à partir d'un plateau unique, adjoindre à ce dernier une quille adaptée à un besoin spécifique à partir d'un jeu de quilles à sa disposition. Il en découle que la prothèse de l'invention est compétitive en raison du fait que des quilles de tailles différentes peuvent être fabriquées indépendamment du plateau, un même plateau pouvant être utilisé pour différentes quilles.

Selon un deuxième aspect de l'invention, l'autre élément d'emboîtement de la prothèse est du genre d'élément comportant une section longitudinale globalement conformée en segment circulaire. Son organe de positionnement est constitué par au moins un rail, quille ou équivalent, prévu pour être emboîté à l'intérieur d'un évidemment, tel qu'une saignée en corrélation avec le rail, ménagé dans l'os correspondant, astragale notamment, pour le centrage sur ce dernier de l'élément d'emboîtement, dit astragalien, dans le cas d'exemple d'une prothèse de la cheville.

Selon une forme préférée de réalisation de l'élément astragalien, celui-ci comporte au moins un plan d'appui transversal à l'encontre d'un glissement longitudinal de l'élément astragalien. Ce plan d'appui est ménagé sur la face inférieure et à l'arrière de l'élément astragalien, et est préférentiellement incliné d'un angle C, de manière à former un dièdre entre les deux plans, pour limiter les aménagements correspondant de l'astragale. Il en résulte que l'appui à faible portée de l'élément astragalien dans le sens longitudinal que procure l'emboîtement du rail à l'intérieur de l'astragale, est conforté par sa mise en appui longitudinal étendu par l'intermédiaire du plan d'appui qu'il comporte.

Pour préparer la surface de l'astragale prévue pour recevoir le plan d'appui longitudinal de l'élément astragalien, il est proposé un instrument spécifique comportant, en bout d'un manche de préhension, une platine pourvue d'au moins un organe de positionnement sur l'astragale et une fenêtre pour le passage et le guidage d'un outil de préparation de la dite surface.

On comprendra que l'organe de positionnement de la platine est avantageusement similaire à celui de positionnement de l'élément astragalien sur l'astragale et est prévu pour être logé à l'intérieur de l'évidemment correspondant préalablement réalisé par le praticien. Il en découle que la préparation de la surface d'appui longitudinal de l'élément astragalien s'effectue en correspondance identique avec la configuration de ce dernier au regard de son centrage sur l'astragale.

La fenêtre pour le passage et le guidage de l'outil est par exemple ménagée à travers un retour de la platine, et est avantageusement inclinée par rapport à sa face de repos sur l'astragale d'un angle D correspondant à celui C d'inclinaison du plan d'appui longitudinal de l'élément astragalien sur l'astragale. Préférentiellement de conformation oblongue, sa longueur est de l'ordre de la largeur de la face de l'astragale à préparer.

Selon un autre aspect de l'invention, il est proposé un instrument pour le centrage des éléments tibial et astragalien l'un par rapport à l'autre. Cet instrument, à la manière connue, comporte en bout d'un manche de préhension une platine pourvue d'au moins une lumière pour le passage d'un outil, destiné à pratiquer dans l'os correspondant l'évidemment prévu pour recevoir l'organe de positionnement de l'élément astragalien. Selon l'invention, la platine est équipée en outre d'un organe de positionnement prévu pour coopérer avec un organe complémentaire ménagé à l'intérieur du plateau d'un élément tibial, d'essai notamment, préalablement centré sur l'os correspondant. Il en découle que le positionnement de l'évidemment prévu pour recevoir l'organe de centrage de l'élément astragalien est pratiqué corrélativement au centrage de l'élément tibial à l'intérieur de l'os correspondant. On relèvera que selon cet aspect de l'invention, l'élément tibial comporte à sa base un organe de positionnement d'une platine participante d'un instrument correspondant de l'invention. On comprendra aussi que le positionnement de la lumière au moins par rapport à l'élément tibial est tant un positionnement transversal par rapport à l'axe d'extension de la quille, qu'un positionnement de son orientation par rapport à l'astragale.

Par exemple, l'organe de positionnement de la platine est constitué d'un téton prévu pour pénétrer dans un orifice ménagé à la base du plateau de l'élément tibial, celui-ci étant de préférence globalement coaxial à l'axe général d'extension de la quille. Le téton est quant à lui positionné sur la platine en conséquence.

Selon une forme préférée de réalisation, la platine comporte en outre au moins un organe d'ancrage à l'intérieur de l'os correspondant, constitué par exemple par un picot en vue d'interdire un déplacement inopiné de la platine pendant l'opération de préparation de l'évidemment prévu pour recevoir l'organe de positionnement de l'élément astragalien. On notera que dans le cas où l'organe de positionnement de la platine par rapport à l'élément tibial est un organe de révolution, tel que le téton susvisé, l'organe d'ancrage est décalé par rapport à l'axe d'extension de l'organe de révolution pour permettre le maintien de la platine à l'encontre de son pivotement.

Avantageusement, la platine et l'organe de positionnement de cette dernière sur l'élément tibial sont ménagés à l'extrémité d'une branche respective d'une pince, à crémaillère notamment, d'écartement des os de l'articulation.

On comprendra que les dispositions susvisées relatives à l'instrument de centrage des éléments tibial et astragalien l'un par rapport dans le cas d'exemple d'une prothèse de la cheville, sont applicables d'une part à toute prothèse d'articulation composée d'éléments prévus pour être reliés à un os respectif, et d'autre part à une prothèse de la cheville indépendamment des caractéristiques susvisées de l'invention relatives à chacun de ces éléments.

La présente invention sera mieux comprise et des détails en relevant apparaîtront, à la description qui va en être faite d'une forme préférée de réalisation, en relation avec les figures des planches annexées, dans lesquelles :
les fig.1 et fig.2 sont des illustrations en perspective d'une prothèse selon une forme préférée de réalisation de l'invention, respectivement en vue éclatée et en vue assemblée.
la fig.3 est une vue en coupe éclatée d'un élément tibial participant d'une prothèse illustrée sur les fig. 1 et fig.2,
la fig.4 est une vue en perspective d'une quille participant d'un élément tibial illustré sur la fig.3.
les fig.5 et fig.6 sont des illustrations respectivement vue de côté et vue de dessus d'un instrument pour le centrage des éléments tibial et astragalien l'un par rapport à l'autre, selon une forme préférée de réalisation,
les fig.7 et fig.8 sont des illustrations en vues partielles respectivement en coupe et de dessus d'un instrument pour la préparation d'une face de l'astragale prévue pour l'appui longitudinal de l'élément astragalien, selon une forme préférée de réalisation.

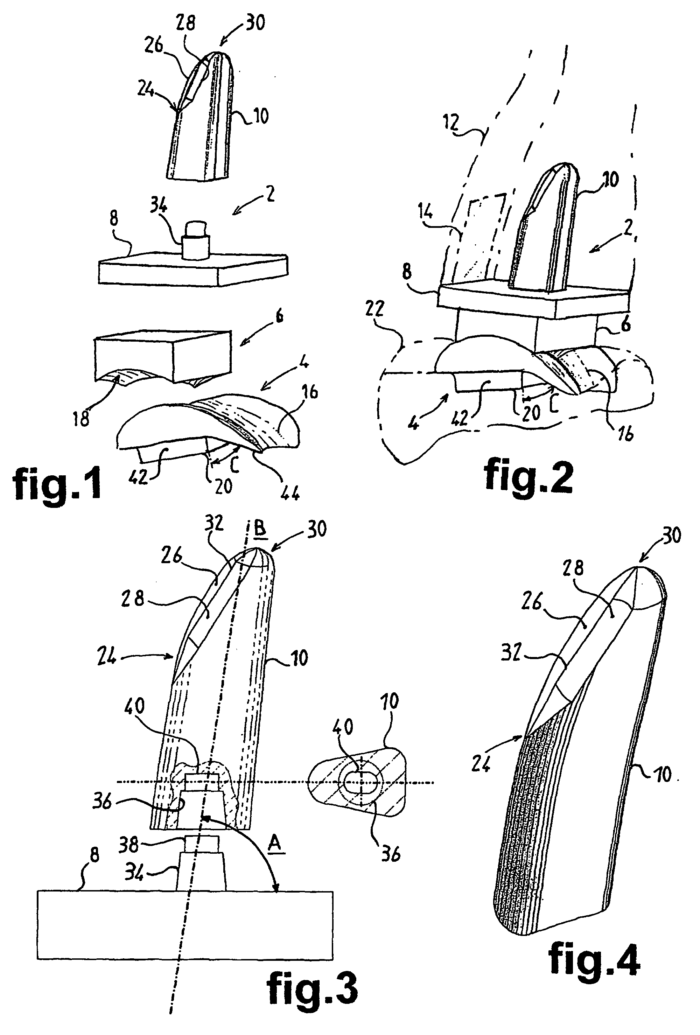

Sur les fig.1 et fig.2, une prothèse de la cheville est composée d'un élément tibial 2 et d'un élément astragalien 4 entre lesquels est interposé un élément intermédiaire 6 pour leur articulation relative.

L'élément tibial 2 comporte un plateau 8 situé à son extrémité proximale, prévu pour reposer sur la face supérieure de l'élément intermédiaire 6. Il comporte en outre une quille 10 à son extrémité distale, prévue pour être introduite à l'intérieur d'un logement du tibia 12 ménagé extemporanément par le praticien, en vue du centrage de l'élément tibial 2. On notera la présence d'une fenêtre frontale 14 pratiquée dans le tibia 12 pour faciliter le mise en place de l'élément tibial 2.

L'élément astragalien 4 est d'une section longitudinale globalement conformée en segment circulaire, et comporte quant à lui à son extrémité proximale une face supérieure 16 longitudinalement convexe prévue pour coopérer avec une face inférieure concave 18 de l'élément intermédiaire 6. On remarquera que ces surfaces de contact 16 et 18, entre l'élément astragalien 4 et l'élément intermédiaire 6, sont aussi transversalement incurvées pour leur positionnement relatif transversal.

Sur les fig.1 à fig.4, et selon un premier aspect de l'invention, la quille 10 de l'élément tibial 2 comporte une zone médiane d'inflexion 24 vers son extrémité distale. Cette inflexion 24 forme une surface 26,28 de prise de la quille 10 en vue de l'extraction de l'élément tibial 2. Cette surface de prise 26,28 est inclinée vers le plateau 8 par rapport à la surface extérieure d'extension générale de la quille 10. En outre, l'axe général B d'extension de la quille 10 est lui-même inclinée par rapport à l'orientation générale du plateau, d'un angle A de l'ordre de 85°.

En se reportant plus particulièrement sur la fig.4, la surface de prise 26,28 est conformée en au moins un méplat de la surface extérieure de la quille 10. Plus particulièrement, la surface de prise 26,28 s'étend sur la zone d'inflexion 24 de la quille 10, c'est à dire depuis sa zone médiane jusqu'à son extrémité distale 30, ou sommet. La surface de prise 26,28 est composée d'une pluralité de facettes ménagées sur la surface extérieure de la quille 10, dont au moins une facette frontale 26 avoisinée de deux facettes latérales 28.

On comprendra par facette frontale 26 une facette ménagée sur la quille 10 dans sa zone prévue pour être en regard sur la fenêtre tibiale 14 opérée par le praticien. On remarquera que la quille 10 comporte une section globalement triangulaire à arrêtes arrondies. Sur l'exemple illustré, la facette frontale 26 s'étend sur la face frontale de la quille 10, et les facettes latérales 28 s'étendent depuis leur extrémité proximale vers leur extrémité distale, sur la face frontale et une face latérale respective de la quille 10, sans toutefois que leur extension transversale n'excède la moitié des faces latérales de la quille 10. On remarquera donc que de préférence, les arrêtes communes, telles que 32, entre celles des facettes latérales 28 et celles de la facette frontale 26 ont une orientation concourante et tendent à se rejoindre à chacune des extrémités des facettes 26,28.

En d'autres termes, les facettes frontale 26 et latérales 28 sont effilées à leurs extrémités distales et proximales, pour se rejoindre respectivement en zone médiane de la quille 10 et au sommet 30 de cette dernière.

Sur la fig.3, la quille 10 et le plateau 8 sont reliés l'un à l'autre par l'intermédiaire d'un cône d'emboîtement 34 que comporte le plateau 8, prévu pour pénétrer en contact étroit à l'intérieur d'un évidemment correspondant 36 de la quille 10. On notera que cet emboîtement est complété par un emboîtement non circulaire entre un organe mâle 38 du plateau 8 et un logement correspondant 40 de la quille 10, pour immobiliser cette dernière en rotation par rapport au plateau 8.

En revenant aux fig.1 et fig.2, l'astragale 22 est extemporanément préparée par le praticien, de manière à ce que son dôme puisse recevoir la base 20 de l'élément astragalien 4. Des saignées sont extemporanément ménagées dans l'astragale 22 pour loger des rails 42 de centrage de l'élément astragalien 4. Ce dernier comporte en outre une surface inclinée 44 formant un plan d'appui longitudinal de l'élément astragalien sur l'astragale. On comprendra que cette inclinaison C est relative à la surface de base 20 de l'élément astragalien.

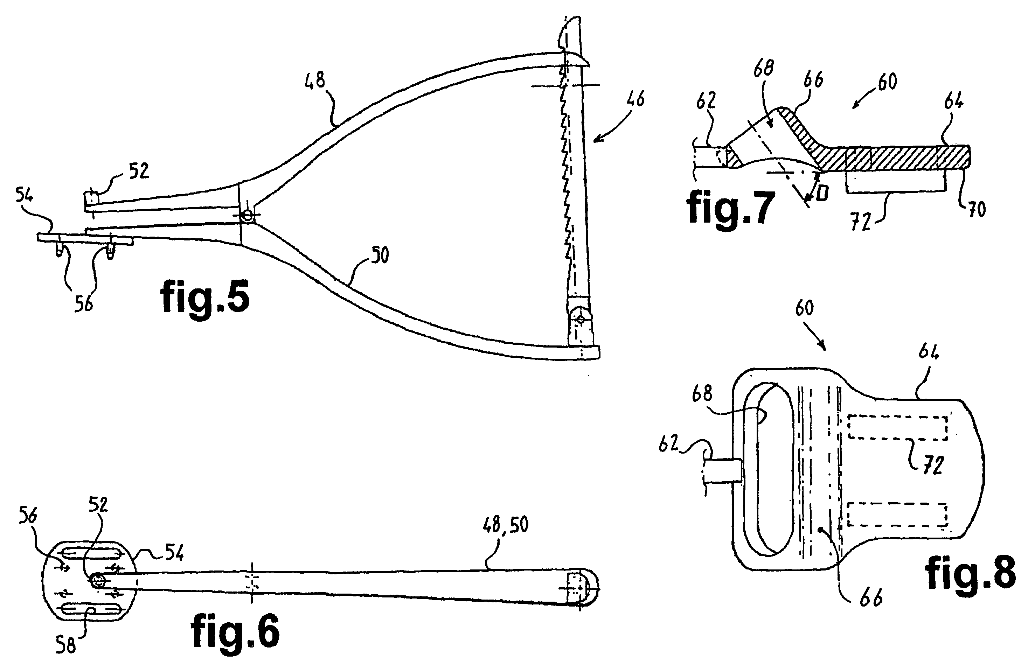

Sur les fig.5 et fig.6, une pince à crémaillère 46 est utilisée pour écarter le tibia 12 de l'astragale 22 en vue de l'implantation de la prothèse. Les branches 48 et 50 de la pince 46 comportent en bout distal un téton 52 pour l'une (48) et une platine 54 pour l'autre (50). Le téton 52 est prévu pour être logé dans un évidemment correspondant d'un élément tibial d'essai, tandis que la platine 54 est prévue pour être ancrée à l'intérieur de l'astragale par l'intermédiaire de picots 56 dont elle est pourvue. Grâce à ces dispositions, la platine 54 est centrée sur l'astragale par rapport à l'élément tibial 2, et le praticien peut opérer dans l'astragale 22 les saignées prévues pour recevoir les rails 42 de l'élément astragalien 4. On remarquera à travers la platine 54 la présence de lumières 58 pour le passage de l'outil habituellement utilisé pour ménager les saignées dans l'astragale 22.

Sur les fig.7 et fig.8, un instrument 60 est utilisé pour préparer la face arrière de l'astragale 22, contre laquelle l'élément astragalien 4 prend un appui longitudinal. Cet instrument comprend un manche 62 en bout duquel est supportée une platine 64 pourvue d'un retour 66. Pour son centrage sur l'astragale 22, la platine 64 comporte des rails 72 similaires à ceux de l'élément astragalien 4, qui sont prévus pour être logés à l'intérieur des saignées y relatives préalablement opérées par le praticien dans l'astragale 22. Le retour 66 comporte quant à lui à son travers une fenêtre 68 pour le passage d'un outil de préparation de la face arrière de l'astragale 22. On remarquera que la fenêtre 68 est inclinée par rapport à la base 70 de la platine 60 prévue pour reposer sur l'astragale 22, d'un angle D correspondant à l'angle C d'inclinaison du plan d'appui longitudinal 44 de l'élément astragalien 4.

## Revendications

1. Prothèse d'articulation entre deux os (12,22), prothèse de la cheville notamment, du genre de prothèse comprenant deux éléments d'emboîtement (2,4) prévus pour être introduits à l'intérieur d'un logement d'un os respectif (12,22), et un élément intermédiaire (6) interposé entre les éléments d'emboîtement pour leur articulation l'un par rapport à l'autre et leur mise en compression contre l'os correspondant, les éléments d'emboîtement comportant chacun un organe distal (10,42) de positionnement à l'intérieur de l'os y relatif et un organe proximal (8,16) d'appui contre une face opposée respective de l'élément intermédiaire (6), l'organe distal (10) de positionnement, dit quille, de l'un des éléments d'emboîtement (2), dit tibial, étant un organe de centrage d'une conformation allongée globalement conique se réduisant vers son extrémité distale, prévu pour pénétrer axialement à l'intérieur de l'os correspondant (12), tibia notamment, l'organe proximal (8) d'appui de l'élément tibial (2) contre la face correspondante de l'organe intermédiaire (6) étant quant à lui conformé en plateau relié à l'extrémité proximale de la quille avec une orientation générale sensiblement perpendiculaire à l'axe B d'extension de cette dernière (10), **caractérisé :**
**en ce que** la quille (10) comporte des moyens (24,26,28,A), dits de prise, pour sa prise et son extraction par le praticien hors du logement qui la reçoit, les dits moyens (24,26,28,A) comprenant au moins une zone d'inflexion (24) médiane de la quille (10) vers son extrémité distale pour former une surface (24,26), dite de prise, inclinée par rapport à la surface extérieure d'extension générale de la quille (10) depuis son extrémité proximale,
de telle sorte que le praticien puisse prendre fermement appui contre la surface de prise (24,26) au moyen d'un outil, et exercer une traction sur la quille (10) vers son extrémité proximale en vue de son extraction hors du logement qui la reçoit.

2. Prothèse selon la revendication 1, **caractérisée :**
**en ce que** les moyens de prise (24,26,28,A) de la quille comprennent au moins une conformation globalement plane de la surface de prise (26,28), qui est formée par au moins une facette (24,26) ménagée sur la surface extérieure de la quille (10).

3. Prothèse selon l'une quelconque des revendications précédentes, **caractérisée:**
**en ce que** les moyens de prise (24,26,28,A) de la quille (10) comprennent une inclinaison générale A de la quille 10 par rapport au plateau (8), de manière à accroître l'inclinaison de la surface de prise (26,28) par rapport à l'orientation générale de ce dernier (8).

4. Prothèse selon l'une quelconque des revendications précédentes, **caractérisée:**
**en ce que** les moyens de prise (24,26,28,A) de la quille (10) comprennent une conformation tronc pyramidale de la quille (10), à section globalement triangulaire à arrêtes arrondies .

5. Prothèse selon les revendications 2 et 4, **caractérisée:**
**en ce que** la surface de prise (26,28) est composée d'une pluralité de facettes (26,28) ménagées sur la surface extérieure de la quille (10), dont au moins une facette frontale (26) avoisinée de deux facettes latérales (28), les facettes frontales (26) et latérales (28) étant effilées à leurs extrémités distales et proximales, pour se rejoindre respectivement en zone médiane de la quille (10) et au sommet (30) de cette dernière (10).

6. Prothèse selon l'une quelconque des revendications précédentes, **caractérisée:**
**en ce que** les moyens de liaison (34,38,36,40) entre la quille (10) et le plateau (8) sont des moyens de liaison par emboîtement constitués par un organe mâle (34,38) et un organe femelle (36,40) coopérant, respectivement ménagés sur l'un quelconque du plateau (8) et de la quille (10),
de telle sorte que le praticien puisse, à partir d'un plateau (8) unique, adjoindre à ce dernier (8) une quille (10) adaptée à un besoin spécifique à partir d'un jeu de quilles (10) à sa disposition.

7. Prothèse selon l'une quelconque des revendications précédentes, l'autre des éléments d'emboîtement (4), dit astragalien, comportant une section longitudinale globalement en segment circulaire, et un organe de positionnement (42) pour son centrage sur l'os correspondant (22), astragale notamment, **caractérisé :**
**en ce que** l'élément astragalien (4) comporte au moins un plan d'appui transversal (44) à l'encontre de son glissement longitudinal, ménagé sur la face inférieure (20) et à l'arrière de l'élément astragalien (4),
de telle sorte que l'appui de l'élément astragalien (4) dans le sens longitudinal que procure son organe de centrage (42) à l'intérieur de l'os correspondant (22), est conforté par sa mise en appui longitudinal étendu par l'intermédiaire du plan d'appui (44) qu'il comporte.

8. Prothèse selon la revendication 7, **caractérisée :**
**en ce que** le dit plan d'appui (44) est incliné d'un angle C pour limiter les aménagements correspondant de l'astragale (22).

9. Instrument pour la préparation de la surface de l'os (22) prévue pour recevoir le plan d'appui longitudinal (44) de l'élément astragalien (4) participant d'une prothèse selon l'une quelconque des revendications 7 et 8, **caractérisé :**
**en ce qu'**il comporte, en bout d'un manche (62) de préhension, une platine (60) pourvue d'au moins un organe de positionnement (72) sur l'astragale (22) et une fenêtre (68) pour le passage et le guidage d'un outil de préparation de la dite surface.

10. Instrument selon la revendication 9, **caractérisé :**
**en ce que** l'organe de positionnement (72) de la platine (60) est similaire à celui (42) de positionnement de l'élément astragalien (4) sur l'astragale (22), et est prévu pour être logé à l'intérieur de l'évidemment correspondant préalablement réalisé par le praticien,
de telle sorte que la préparation de la surface d'appui longitudinal de l'élément astragalien (4) s'effectue en correspondance identique avec la configuration de ce dernier au regard de son centrage sur l'astragale (22).

11. Instrument selon l'une quelconque des revendications 9 et 10, pour la préparation de la surface de l'os (22) prévue pour recevoir le plan d'appui longitudinal de l'élément astragalien (4) participant d'une prothèse selon la revendication 8, **caractérisé :**
**en ce que** la dite fenêtre (68) est ménagée à travers un retour (66) de la platine (60) et est inclinée par rapport à sa face de repos (70) sur l'astragale (22) d'un angle D correspondant à celui C d'inclinaison du plan d'appui longitudinal (44) de l'élément astragalien (4) sur l'astragale (22).

12. Instrument pour le centrage l'un par rapport à l'autre des éléments tibial (2) et astragalien (4) participant d'une prothèse selon l'une quelconque des revendications 7 à 8, du genre d'instrument comportant en bout d'un manche de préhension (48,50) une platine (54) pourvue d'au moins une lumière (58) pour le passage d'un outil, destiné à pratiquer dans l'os correspondant (22) l'évidemment prévu pour recevoir l'organe de positionnement (42) de l'élément astragalien (4), **caractérisé :**
**en ce que** la platine (54) est équipée d'un organe de positionnement (52) prévu pour coopérer avec un organe complémentaire ménagé à l'intérieur du plateau (8) de l'élément tibial (2), préalablement centré sur l'os correspondant (12),
de telle sorte que le positionnement de l'évidemment prévu pour recevoir l'organe de centrage (42) de l'élément astragalien (4) est pratiqué corrélativement au centrage de l'élément tibial (2) à l'intérieur de l'os correspondant (12).

13. Instrument selon la revendication 12, **caractérisé :**
**en ce que** l'organe de positionnement (52) de la platine (54) est constitué d'un téton prévu pour pénétrer dans un orifice ménagé à la base du plateau (8) de l'élément tibial (2).

14. Instrument selon l'une quelconque des revendications 12 et 13, **caractérisé :**
**en ce que** la platine (54) comporte au moins un organe d'ancrage (56) à l'intérieur de l'os correspondant (22) en vue d'interdire un déplacement inopiné de la platine (54) pendant l'opération de préparation de l'évidemment prévu pour recevoir l'organe de positionnement (42) de l'élément astragalien (4).

15. Instrument selon la revendication 14, **caractérisé :**
**en ce que** l'organe de positionnement (52) de la platine (54) par rapport à l'élément tibial (2) étant un organe de révolution, le dit organe d'ancrage (56) est décalé par rapport à l'axe d'extension du dit organe de révolution (52) pour favoriser le maintien de la platine (54) à l'encontre de son pivotement.

16. Instrument selon l'une quelconque des revendications 12 à 15, **caractérisé :**
**en ce que** la platine (54) et l'organe de positionnement (52) de cette dernière sur l'élément tibial (2) sont ménagés à l'extrémité d'une branche respective (48,50) d'une pince (46) d'écartement des os (12,22) de l'articulation.

## Claims

1. Articulation prosthesis between two bones (12, 22), in particular prosthesis of the ankle, of the type comprising two interlocking elements (2, 4) designed to be inserted into a housing of a respective bone (12, 22), and an intermediate element (6) inserted between the interlocking elements for their articulation of one in relation to the other, and their compression against the corresponding bone, wherein the interlocking elements each comprise a distal positioning mechanism (10, 42) inside the associated bone, and a proximal mechanism (8, 16) for support against a respective opposing face of the intermediate element (6), wherein the positioning distal mechanism (10), the so-called pin, of one of the interlocking elements (2), so-called tibial element, is a mechanism centring a generally conical elongated conformation that tapers towards its distal end, designed to penetrate axially inside the corresponding bone (12), in particular the tibia, and wherein the proximal mechanism (8) supporting the tibial element (2) against the corresponding face of the intermediate mechanism (6) is shaped to form a plate connected to the proximal end of the pin, in a general orientation that is essentially perpendicular to extension axis B of the latter (10), **characterised:**
**in that** the pin (10) comprises means (24, 26, 28, A), so-called socket, for its gripping and extraction by the practitioner outside the housing receiving it, wherein the said means (24, 26, 28, A) comprise at least one median zone of inflection (24) of the pin (10) toward its distal end to form a so-called gripping surface (24, 26), inclined in relation to the outer surface of general extension of the pin (10) from its proximal end,
so that the practitioner is able to gain firm support against the socket surface (24, 26) using a tool, and exert tractive force on the pin (10) toward its proximal end in order to extract from the housing receiving it.

2. Prosthesis according to Claim 1, **characterised:**
**in that** the means (24, 26, 28, A) for gripping the pin include at least one generally flat conformation of the gripping surface (26, 28), which is formed by at least one facet (24, 26) arranged on the outer surface of the pin (10).

3. Prosthesis according to any of the preceding claims, **characterised:**
**in that** the means (24, 26, 28, A) for gripping the pin (10) include a general inclination A of the pin 10 in relation to the plate (8) so that the inclination of the gripping surface (26, 28) is increased in relation to the general orientation of the latter (8).

4. Prosthesis according to any of the preceding claims, **characterised:**
**in that** the means (24, 26, 28, A) for gripping the pin (10) include a truncated pyramid conformation of the pin (10), with an overall triangular section with rounded edges

5. Prosthesis according to Claims 2 and 4, **characterised:**
**in that** the gripping surface (26, 28) consists of a plurality of facets (26, 28) arranged on the outer surface of the pin (10), at least one frontal facet (26) of which is adjacent to two lateral facets (28), wherein the front (26) and lateral (28) facets are tapered at their distal and proximal ends and converging again in the median zone of the pin (10) and at the vertex (30) of the latter (10).

6. Prosthesis according to any of the preceding claims, **characterised:**
**in that** the means of connection (34, 38, 36, 40) between the pin (10) and the plate (8) are means of connection by interlocking, comprising an interacting male mechanism (34, 38) and female mechanism (36, 40) arranged on either the plate (8) or the pin (10),
so that the practitioner is able, from a single plate (8), to add to the latter (8) a pin (10) adapted to a specific requirement from a set of pins (10) at his disposal.

7. Prosthesis according to any of the preceding claims, wherein the other interlocking element (4), so-called astragalar element, comprising a longitudinal section essentially in a circular segment, and a positioning mechanism (42) for centring it on the corresponding bone (22), in particular the astragalar bone, **characterised:**
**in that** the astragalar element (4) includes at least one transversal plane of support (44) preventing its longitudinal sliding, arranged on the lower face (20) and behind the astragalar element (4),
so that the support of the astragalar element (4) in the longitudinal direction assumed by its centring mechanism (42) inside the corresponding bone (22) is strengthened by its longitudinal support extended by means of the plane of support (44) which it includes.

8. Prosthesis according to Claim 7, **characterised:**
**in that** the said plane of support (44) is inclined at an angle C to limit the corresponding adjustments of the astragalus (22).

9. Device for preparation of the surface of the bone (22) designed to receive the longitudinal plane of support (44) of the astragalar element (4) forming part of a prosthesis according to any of Claims 7 and 8, **characterised:**
**in that** it comprises, at the end of a gripping sleeve (62), a plate (60) provided with at least one positioning mechanism (72) on the astragalus (22) and a window (68) for passing through and guiding a tool for preparing the said surface.

10. Device according to Claim 9, **characterised:**
**in that** the positioning mechanism (72) of the plate (60) is similar to the positioning mechanism (42) of the astragalar element (4) on the astragalus (22), and is designed to be housed inside the corresponding cavity previously made by the practitioner,
so that the preparation of the longitudinal supporting surface of the astragalar element (4) is carried out to correspond exactly to the configuration of the latter relative to its centring on the astragalus (22).

11. Device according to any of Claims 9 and 10 for preparation of the surface of the bone (22), designed to receive the longitudinal plane of support of the astragalar element (4) forming part of a prosthesis according to Claim 8, **characterised:**
**in that** the said window (68) is arranged across a return (66) of the plate (60) and is inclined in relation to its surface of rest (70) on the astragalus (22)

12. Device for centring one in relation to the other of the tibial (2) and astragalar (4) elements constituting a prosthesis according to any of Claims 7 to 8, of the type of device comprising, at the end of a gripping sleeve (48, 50), a plate (54) provided with at least one opening (58) for passing through a tool, designed to make in the corresponding bone (22) the cavity designed to receive the positioning mechanism (42) of the astragalar element (4), **characterised:**
**in that** the plate (54) is fitted with a positioning mechanism (52) designed to interact with a supplementary mechanism arranged on the inside of the plate (8) of the tibial element (2) previously centred on the corresponding bone (12),
so that the positioning of the cavity provided to receive the centring mechanism (42) of the astragalar element (4) is made in correlation with the centring of the tibial element (2) inside the corresponding bond (12).

13. Device according to Claim 12, **characterised:**
**in that** the positioning mechanism (52) of the plate (54) consists of a stud designed to penetrate an opening arranged at the base of the plate (8) of the tibial element (2).

14. Device according to any of Claims 12 and 13, **characterised:**
**in that** the plate (54) comprises at least one anchoring mechanism (56) inside the corresponding bone (22) in order to prevent an unexpected displacement of the plate (54) during the operation of preparing the cavity provided for receiving the positioning mechanism (42) of the astragalar element (4).

15. Device according to Claim 14, **characterised:**
**in that** since the positioning mechanism (52) of the plate (54) for positioning it relative to the tibial element (2) is a revolving element, the said anchoring mechanism (56) is offset in relation to the axis of extension of the said revolving mechanism (52), thus helping to prevent the plate (54) from pivoting.

16. Device according to any of Claims 12 to 15, **characterised:**
**in that** the plate (54) and the positioning mechanism (52) for positioning the latter on the tibial element (2) are arranged at the end of a respective branch (48, 50) of pincers (46) for separating the bones (12, 22) of the joint.

## Patentansprüche

1. Gelenkprothese zwischen zwei Knochen (12,22), vorwiegend Knöchelprothese eines Prothesentyps bestehend aus zwei aufsteckbaren Elementen (2,4), die zur Einführung ins Innere des jeweiligen Knochengehäuses vorgesehen sind (12,22), und einem Zwischenelement (6), das im gegenseitigen Verhältnis zum Gelenk und zur Stauchung gegen den entsprechenden Knochen zwischen die aufsteckbaren Elemente gelegt wird, jedes der aufsteckbaren Elemente besteht aus einem distalen Positionsteil (10,42) zur Positionierung im Inneren des dazugehörigen Knochens und einem proximalen Teil (8,16) zum Halt gegen die jeweils dem Zwischenelement (6) gegenüberliegende Seite, das distale Positionsteil (10), Kegel genannt, eines der aufsteckbaren Elemente (2), Schienbein genannt, ist ein Zentrierungsteil eines verlängerten, insgesamt konischen Aufbaus, der sich zu seinen distalen Endpunkten hin verringert, vorgesehen zum mittigen Einfahren ins Innere des entsprechenden Knochens (12), vor allem ins Schienbein, das proximale Stützteil (8) des Schienbeinelements (2) gegen die gegenüberliegende Seite des Zwischenteils (6) ist diesbezüglich passend zum Teller, der mit dem proximalen Endpunkt des Kegels mittels einer deutlich senkrechten Hauptausrichtung zur Achse B der Erweiterung der letzteren (10) verbunden ist, **gekennzeichnet durch**:
der Kegel (10), enthält Hilfsmittel (24,26,28,A), Griffe genannt, zu dessen Halt und Abstreifen **durch** den Arzt außerhalb des Gehäuses, das er abnimmt, besagte Hilfsmittel (24,26,28,A) bestehen aus mindestens einer Beugezone (24) in der Mitte des Kegels (10), der auf seinen distalen Endpunkt zuläuft, um die Oberfläche (24,26) besagter Griffe zu bilden, geneigt im Verhältnis zur äußeren Oberfläche der Haupterweiterung des Kegels (10) von dessen proximalem Endpunkt,
so dass der Arzt mittels eines Werkzeugs einen festen Halt gegen die Oberfläche des Griffes (24,26) geben, und einen Zug auf den Kegel (10) in Richtung seines proximalen Endpunktes ausüben kann, mit dem Ziel der Abnahme außerhalb des Gehäuses.

2. Prothese gemäß Patentanspruch 1, **gekennzeichnet durch**:
die Griffmittel (24,26,28,A) des Kegels bestehen aus mindestens einem insgesamt glatten Oberflächenaufbau des Griffs (26,28), der aus mindestens einer Facette (24,26) gebildet wird und auf der äußeren Oberfläche des Kegels (10) angeordnet ist.

3. Prothese gemäß eines beliebigen früheren Patentanspruchs, **gekennzeichnet durch**:
die Griffmittel (24,26,28,A) des Kegels (10) bestehen aus einer generellen Schrägstellung A des Kegels 10 im Verhältnis zum Teller (8), um die Schrägstellung der Griffoberfläche (26,28) im Verhältnis zur Hauptrichtung des letzteren (8) zu erhöhen.

4. Prothese gemäß eines beliebigen früheren Patentanspruchs, **gekennzeichnet durch**:
die Griffmittel (24,26,28,A) des Kegels (10) bestehen aus einem pyramidenförmigen Rumpfaufbau des Kegels (10), mit einem insgesamt dreieckigen Abschnitt mit abgerundetem Anschlag.

5. Prothese gemäß den Patentansprüchen 2 und 4, **gekennzeichnet durch**:
die Oberfläche des Griffs (26,28) ist zusammengesetzt aus einer Vielzahl von Facetten (26,28) angeordnet auf der äußeren Oberfläche des Kegels (10), von denen mindestens eine Stirnfacette (26) von zwei seitlichen Facetten (28) begrenzt wird, die Stimfacette (26) und die seitlichen Facetten (28) laufen spitz auf ihre distalen und proximalen Endpunkte zu, und stoßen im mittleren Bereich des Kegels (10) und auf der Spitze (30) des letzteren (10) wieder aufeinander.

6. Prothese gemäß eines beliebigen früheren Patentanspruchs, **gekennzeichnet durch**:
die Verbindungsmittel (34,38,36,40) zwischen dem Kegel (10) und dem Teller (8) sind aufsteckbare Verbindungsmittel bestehend aus einem männlichen (34,38) und einem weiblichen (36,40) Teil, die zusammenarbeiten beziehungsweise beliebig auf dem Teller (8) des Kegels (10) angeordnet sind,
so dass ein Arzt von einem einheitlichen Teller (8) aus letzterem (8) einen Kegel (10) hinzufügen kann, der aus einem ihm zur Verfügung stehenden Satz Kegel (10) auf den speziellen Bedarf angepasst wird.

7. Prothese gemäß eines beliebigen früheren Patentanspruchs, das andere der aufsteckbaren Elemente (4), Astragalien genannt, bestehend aus einem ganzen Längsabschnitt im Kreissegment, und einem Positionsteil (42) für die Zentrierung des betreffenden Knochens (22), des Talus im Besonderen, **gekennzeichnet durch**:
das Astragalien-Element (4) besteht aus mindestens einer seitlichen Auflagefläche (44) angeordnet gegen seine Längsverschiebung, auf der Innenseite (21) und auf der Rückseite des Astragalien-Elements (4).
so dass die Auflage des Astragalien-Elements (4) in Längsrichtung zu den mittleren Teilen (42) im Inneren des betreffenden Knochens (22) führt, verstärkt **durch** seine Längsauflage, erweitert **durch** eine mittlere Auflagefläche (44), mit der das Element ausgestattet ist.

8. Prothese gemäß Patentanspruch 7, **gekennzeichnet durch**:
besagte Auflagefläche (44) ist im C-Winkel geneigt, um die Modifikationen entsprechend des Talus (22) einzuschränken.

9. Vorrichtung zur Vorbereitung der Knochenoberfläche (22) vorgesehen zur Aufnahme der Längsauflagefläche (44) des Astragalien-Elements (4) als Teil einer Prothese gemäß einem der Patentansprüche 7 und 8, **gekennzeichnet durch**:
bestehend aus einem Haltegriff (62), einer Platine (60) versehen mit mindestens einem Positionsteil (72) auf dem Talus (22) und einem Fenster (68) für die Durchführung und die Lenkung einer Vorrichtung zur Vorbereitung besagter Oberfläche.

10. Vorrichtung gemäß Patentanspruch 9, **gekennzeichnet durch**:
das Positionsteil (72) der Platine (60) ist vergleichbar mit jenem (42) der Positionierung des Astragalien-Elements (4) auf dem Talus (22), und ist vorgesehen zur Anbringung im offensichtlich zuvor entsprechend vom Arzt durchgeführten Inneren,
so dass die Vorbereitung der Oberfläche der Längsauflage des Astragalien-Elements (4) in identischer Übereinstimmung mit der Konfiguration des letzteren hinsichtlich seiner Zentrierung auf dem Talus (22) ausgeführt wird.

11. Vorrichtung gemäß einem der Patentansprüche 9 und 10, für die Vorbereitung der Oberfläche der Knochen (22) vorgesehen zur Aufnahme der Längsauflagefläche des Astragalien-Elements (4) Teil einer Prothese gemäß des Patentanspruchs 8, **gekennzeichnet durch**:
besagtes Fenster (68) ist angeordnet **durch** einen Rücklauf (66) der Platine (60) und ist in einem D-Winkel im Verhältnis zu seiner Fußfläche (70) auf dem Talus (22) geneigt, was dem C-Winkel der Neigung der Längsauflagefläche (44) des Astragalien-Elements (4) auf dem Talus (22) entspricht.

12. Vorrichtung zur Zentrierung der Schienbein- (2) und der Astragalien-Elemente (4) jeweils im Verhältnis zueinander als Teil einer Prothese gemäß einem der Patentansprüche 7 bis 8, vom Typ Vorrichtung bestehend aus einem Haltegriff am Endpunkt (48,50) einer Platine (54) ausgestattet mit mindestens einer Einführöffnung (58) zur Durchführung eines Werkzeugs, bestimmt zur Verwendung im entsprechenden Knochen (22), offensichtlich vorgesehen zur Aufnahme des Positionsteils (42) des Astragalien-Elements (4), **gekennzeichnet durch**:
die Platine (54) ist ausgestattet mit einem Positionsteil (52) vorgesehen zur Kooperation mit einem ergänzenden Teil, angeordnet im Tellerinneren (8) des Schienbeinelements (2), das zuvor auf dem entsprechenden Knochen (12) zentriert wurde,
von der Art, dass die Positionierung des offensichtlich für die Aufnahme des Zentrierteils (42) vorgesehenen Astragalien-Elements (4) korrelativ in der Mitte des Schienbein-Elements (2) im Inneren des entsprechenden Knochens (12) angewandt wird.

13. Vorrichtung gemäß Patentanspruch 12, **gekennzeichnet durch**:
das Positionsteil (52) der Platine (54) besteht aus einem Nippel, der zum Eindringen in eine Öffnung vorgesehen ist, die an der Unterfläche des Tellers (8) des Schienbein-Elements (2) angeordnet ist.

14. Vorrichtung gemäß einem der Patentansprüche 12 und 13, **gekennzeichnet durch**:
die Platine (54) besteht aus mindestens einem Verankerungsteil (56) im Inneren des entsprechenden Knochens (22) zur Verhinderung einer unvorhergesehenen Verschiebung der Platine (54) während der Vorbereitungsarbeiten, die offensichtlich für die Aufnahme des Positionsteils (42) des Astragalien-Elements (4) vorgesehen ist.

15. Vorrichtung gemäß des Patentanspruchs 14, **gekennzeichnet durch**:
das Positionsteil (52) der Platine (54) im Verhältnis zum Schienbeinelement (2) ist ein Rotationsteil, das so genannte Verankerungsteil (56) ist gegen seine Schwenkung versetzt im Verhältnis zur Verlängerungsachse besagten Rotationsteils (52) zur Begünstigung der Wartung der Platine (54).

16. Vorrichtung gemäß einem der Patentansprüche 12 bis 15, **gekennzeichnet durch**:
die Platine (54) und das Positionsteil (52) des letzteren auf dem Schienbeinelement (2) sind am äußersten Ende des betreffenden Schenkels (48,50) mit einer Klemme (46) in einem Abstand zu den Gelenksknochen (12,22) angebracht.
